# EUROPÄISCHE PATENTSCHRIFT

(11) **EP 1 924 313 B1**
(45) Veröffentlichungstag und Bekanntmachung des Hinweises auf die Patenterteilung: **07.03.2012**
(21) Anmeldenummer: 06805690.2
(22) Anmeldetag: 12.09.2006
(51) Int. Cl.: A61M 25/06

(54) **MEDIZINISCHES INSTRUMENT**
MEDICAL INSTRUMENT
INSTRUMENT MEDICAL

(30) Priorität: 16.09.2005 DE 102005044468
(43) Veröffentlichungstag der Anmeldung: 28.05.2008
(73) Patentinhaber: Riek, Siegfried, Dr. med., D-78628 Rottweil (DE); Bachmann, Karl-Heinz, 78667 Villingendorf (DE); Gaiselmann, Thomas, D-78667 Villingendorf (DE)
(72) Erfinder: Reiek, Siegfried, 78628 Rottweil (DE); Bachmann, Karl-Heinz, 78667 Villingendorf (DE); Gaiselmann, Thomas, 78667 Villingendorf (DE)
(74) Vertreter: Modrow, Stephanie
(86) Internationale Anmeldenummer: PCT/EP2006/008856
(87) Internationale Veröffentlichungsnummer: WO 2007/031264

(56) Entgegenhaltungen:
- EP-B1- 0 629 382
- WO-A-2005/044357

## Beschreibung

Die Erfindung betrifft ein medizinisches Instrument gemäß dem Oberbegriff des Patentanspruchs 1.

Bei vielen medizinischen Eingriffen wird eine Kanüle in einen Kanal des Körpers des Patienten eingeführt, um in die Wand des Körperkanals einzustechen oder die Wand des Körperkanals zu durchstechen. Die Kanüle kann eine Injektionskanüle sein, um ein Medikament, z. B. ein Anästhetikum in die Wand des Körperkanals zu applizieren. Ein Beispiel für eine solche Anwendung ist die Injektion eines Lokalanästhetikums in die Wand des Cervixkanals bei intrauterinen Eingriffen, wie z. B. beim Einlegen einer Spirale. Ein weiteres Beispiel ist das Einspritzen von Pharmaka, z. B. das Einspritzen von Hyaluron-Säure in das die Harnröhre umgebende Gewebe zur Behandlung der Harninkontinenz oder zur Behandlung des vesikoureteralen Refluxes bei Kindern. Die Kanüle kann auch eine Punktions- oder Biopsie-Kanüle sein, um die Wand des Körperkanals zu durchstoßen und Körperflüssigkeit oder Gewebsproben zu entnehmen. Solche Biopsie-Kanülen können dabei auch als Stanzkanülen ausgebildet sein. Biopsiepunktionen werden beispielsweise zur Gewebsentnahme aus der Prostata transrectal oder transperineal durchgeführt.

Bei der herkömmlichen Technik wird die Kanüle durch den Kanal des Körpers eingeführt und muss dann gegen die Achse des Körperkanals schräg gestellt werden, um mit der distalen Spitze der Kanüle in die Wand des Körperkanals einzustechen. Das Lumen des Körperkanals lässt dabei im Allgemeinen nur einen relativ kleinen Winkel zwischen der Achse des Körperkanals und der Achse der Kanüle zu, so dass nur ein flacher Einstich in die Wand des Körperkanals möglich ist. Außerdem ist aufgrund des kleinen Winkels zwischen der Achse des Körperkanals und der Achse der Kanüle und der unregelmäßigen Gestalt der Wandoberfläche die Einstichstelle in die Wand in axialer Richtung nur ungenau festzulegen.

Das Einlegen einer Intrauterinspirale in die Gebärmutter ist z. B. für die Patientin äußerst schmerzhaft, da das Einführungsgerät für die Intrauterinspirale durch den auf Dehnreize sehr sensiblen inneren Muttermund (os internum)eingeführt wird. Es ist daher vorteilhaft, ein Anästhetikum im Bereich des inneren Muttermunds zu deponieren. Hierzu kann mit einer Injektionskanüle neben der Cervix in das hintere Vaginalgewölbe eingestochen werden. Dies ist problematisch, da der Einstich schmerzhaft ist und die Injektionskanüle über einen relativ großen Weg durch das Gewebe vorgeschoben werden muss, wobei sich in diesem Bereich auch Blutgefäße befinden. Eine weitere Möglichkeit besteht darin, eine möglichst dünne Injektionskanüle in den Cercixkanal einzuführen und das Lokalanästhetikum durch die Wand des Cervixkanals in die Muskulatur des inneren Muttermundes zu injizieren. Dabei können insbesondere die oben erläuterten Probleme auftreten, dass die Injektionskanüle beim Vorschieben im Cervixkanal in die Wand eindringt, da der Cervixkanal innen eine Schleimhaut mit unregelmäßiger Oberfläche aufweist und die Wände praktisch aneinander anliegen. Eine optimale gewünschte Position der Einstichstelle in die Wand des Cervixkanal im Bereich des inneren Muttermundes ist daher kaum möglich. Außerdem ist der Eindringwinkel für das Einstechen der Kanüle durch das mittels eines Spreizspekulums aufgeweitete Lumen der Vagina begrenzt.

Um eine Kanüle in die Wand des Körperkanals einzustechen, ist es daher bekannt, die Kanüle in einem Führungsrohr anzuordnen. Das Führungsrohr weist einen axialen Führungskanal für die Kanüle auf, der am distalen Ende des Führungsrohres abgebogen ist und zu einer seitlich am Umfang des Führungsrohres angeordneten Öffnung verläuft. Beim Vorschieben der Kanüle in dem Führungskanal wird die distale Spitze der Kanüle abgelenkt und tritt seitlich unter einem Winkel gegen die Achse des Führungsrohres durch die Öffnung aus. Die Kanüle kann damit unter dem durch die Öffnung vorgegebenen Winkel gegen die Achse des Führungsrohres in die Wand des Körperkanals des Patienten eingestochen werden. Sind mehrere über den Umfang des Körperkanals verteilte Einstiche erforderlich, so muss das gesamte Instrument um seine Achse gedreht werden, was zu einer ungünstigen Handhabung führt.

Ein mediziniches Instrument gemäss dem Oberbegriff von Anspruch 1 ist aus dem Dokument WO 2005/044357 A bekannt.

Aus der WO 96/35 464 A1 ist ein Instrument bekannt, bei welchem dieses Problem dadurch beseitigt wird, dass in dem Führungsrohr mehrere achsparallele Führungskanäle angeordnet sind, die an ihrem distalen Ende nach außen gebogen sind und sich in über den Umfang des Führungsrohres verteilten verschiedenen Winkelpositionen öffnen. Durch die verschiedenen Führungskanäle können Kanülen vorgeschoben werden, die dann in den entsprechenden Winkelpositionen in die Wand des Körperkanals des Patienten einstechen, ohne dass das Führungsrohr gedreht werden muss. Dieses Instrument ist allerdings aufwendig und das Führungsrohr muss auf Grund der mehrfachen Führungskanäle einen großen Durchmesser aufweisen. Außerdem ist das Instrument wegen der vorgegebenen Anzahl von Führungskanälen nicht flexibel in Bezug auf die Anzahl der Einstiche und deren Winkelverteilung.

Der Erfindung liegt daher die Aufgabe zu Grunde, ein medizinisches Instrument gemäß dem Oberbegriff des Patentanspruchs 1 zur Verfügung zu stellen, welches einfach in der Handhabung und flexibel in den Verwendungsmöglichkeiten ist.

Diese Aufgabe wird erfindungsgemäß gelöst durch ein medizinisches Instrument mit den Merkmalen des Patentanspruchs 1.

Vorteilhafte Ausführungen der Erfindung sind in den Unteransprüchen angegeben.

Das erfindungsgemäße medizinische Instrument weist ein Führungsrohr mit einem axialen Führungskanal auf, der am distalen Ende nach außen abgebogen verläuft und in einer seitlich am Umfang des Führungsrohres angeordneten Öffnung mündet. Durch diesen axialen Führungskanal kann eine Kanüle geschoben werden, deren distale Spitze beim Vorschieben am distalen Ende des Führungskanals aus der Achse des Führungsrohres abgelenkt wird und gegen die Achse des Führungsrohres abgewinkelt seitlich aus dem Führungsrohr austritt, um in die Wand eines Körperkanals des Patienten einzustechen, in welchen das Führungsrohr eingeführt wurde. Um an verschiedenen über den Umfang verteilten definierten Winkelstellungen der Wand des Körperkanals einstechen zu können, ist das Führungsrohr mit seinem proximalen Ende um seine Längsachse drehbar in einem Griffteil gelagert. Dadurch ist eine einfache Handhabung möglich, da der behandelnde Arzt das Griffteil fest in der Hand halten kann, ohne die Position des Griffteils und damit seine Handhaltung ändern zu müssen. Er muss lediglich das Führungsrohr in dem Griffteil drehen, um die Winkelposition der Austrittsöffnung für die Kanüle am distalen Ende des Führungsrohres zu ändern. Die axiale Lage des distalen Endes des Führungsrohres und damit des Einstichpunktes der aus dem Führungsrohr austretenden Kanüle ist durch die Lage des Griffteils festgelegt, so dass die axiale Lage der Einstichstellen in verschiedenen Winkelpositionen einfach und zuverlässig beibehalten werden kann.

Das Führungsrohr weist nur einen axialen Führungskanal auf, so dass der Durchmesser und Querschnitt des Führungsrohres nicht wesentlich größer sein müssen als der Durchmesser und der Querschnitt der eingesetzten Kanüle. Das Führungsrohr kann beliebig um seine Längsachse gedreht werden, so dass Einstiche in jeder beliebigen Winkelposition möglich sind. Dadurch ergibt sich in der Anwendung eine hohe Flexibilität, da die Anzahl der Einstiche und ihre gegenseitige Winkelposition beliebig gewählt werden können.

Vorzugsweise ist an dem in dem Griffteil aufgenommenen proximalen Ende des Führungsrohres eine Markierung vorgesehen, an welcher der behandelnde Arzt erkennen kann, in welcher Drehwinkelposition sich das Führungsrohr und damit die Öffnung für den Austritt der Kanülenspitze befindet.

In einer zweckmäßigen Ausführung ist an dem proximalen Ende des Führungsrohres ein im Wesentlichen radial abstehender Betätigungshebel angebracht, der einerseits ein einfaches feinfühliges Verdrehen des Führungsrohres ermöglicht und andererseits gleichzeitig als Markierung für die Drehwinkelposition dient.

Vorzugsweise ist das Führungsrohr lösbar an dem Griffteil angeordnet, so dass das Instrument für eine gründliche Reinigung und Sterilisation zerlegt werden kann.

In einer zweckmäßigen Ausführung weist das Führungsrohr an seinem proximalen Ende einen Außenbund auf, der in einem Lagerring am distalen Ende des Griffteils drehbar gelagert ist. In diesen Außenbund kann der Betätigungshebel eingesetzt sein, wobei er durch einen Umfangsschlitz des Lagerrings radial hindurch tritt. Der Betätigungshebel dient auf diese Weise sowohl zum Verdrehen des Führungsrohres, zur Anzeige der Drehwinkelposition des Führungsrohres und zur axialen Sicherung des Führungsrohres an dem Griffteil.

In einer zweckmäßigen Ausführung weist das Griffteil eine Aufnahme für eine handelsübliche Spritze auf. Dadurch ergibt sich eine vielseitige Verwendbarkeit des Instruments. Vorzugsweise ist dabei die Spritze in dem Griffteil axial zwischen einer proximalen Position und einer distalen Position verschiebbar. In der proximalen Position befindet sich die distale Spitze der Kanüle der Spritze innerhalb des Führungskanals des Führungsrohres, so dass das Führungsrohr in den Körperkanal des Patienten eingeführt werden kann. In der distalen Position der Spritze ist die Kanüle soweit vorgeschoben, dass ihre distale Spitze seitlich aus dem Kanülenrohr austritt und in die Wand des Körperkanals des Patienten einstechen kann. Eine einfache Handhabung des Instruments ergibt sich dabei dann, wenn die distale Position und die proximale Position jeweils durch einen Anschlag definiert sind. Der distale Anschlag definiert dabei gleichzeitig auch die Länge, um welche das distale Ende der Kanüle aus dem Umfang des Führungsrohres austritt, d. h. die Einstichtiefe der Kanüle in die Wand des Körperkanals des Patienten.

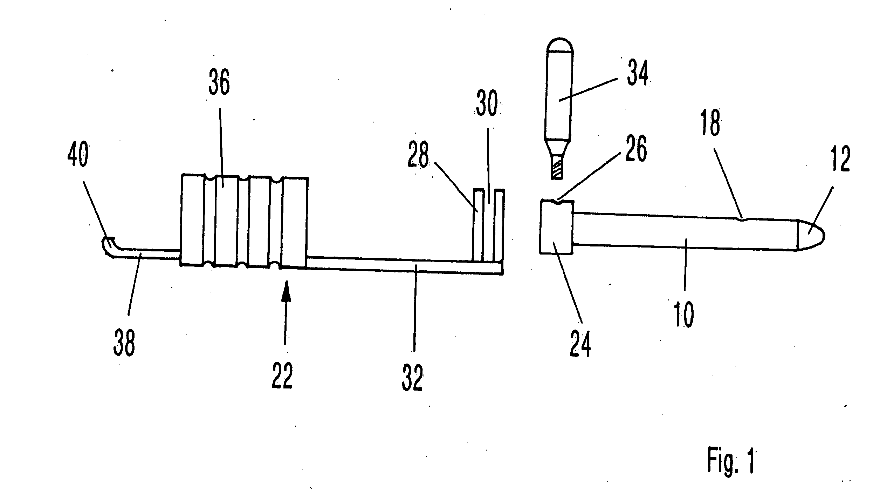

Im Folgenden wird die Erfindung anhand eines in der Zeichnung dargestellten Ausführungsbeispieles näher erläutert. Es zeigen
- Fig. 1: eine Seitenansicht des Instruments in zerlegtem Zustand,

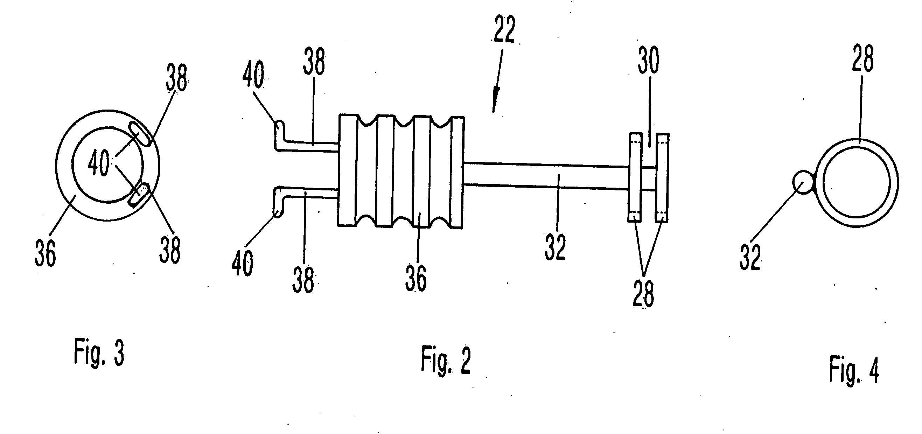

- Fig. 2: eine gegenüber Figur 1 um 90° gedrehte Seitenansicht des Griffteils,
- Fig. 3: eine axiale Stirnansicht auf das in Figur 2 linke proximale Ende des Griffteils,
- Fig. 4: eine axiale Stirnansicht auf das in Figur 2 rechte distale Ende des Griffteils,
- Fig. 5: eine Seitenansicht des Instruments mit eingesetzter Spritze,
- Fig. 6: einen Axialschnitt durch das Führungsrohr mit zurückgezogener Kanüle und
- Fig. 7: einen Figur 6 entsprechenden Axialschnitt mit distal vorgeschobener Kanüle.

Das medizinische Instrument weist ein Führungsrohr 10 auf, welches an seinem distalen Ende 12 geschlossen ist und eine stumpfe abgerundete Spitze aufweist, die gegebenenfalls auch leicht konisch ausgebildet sein kann. Das innere Lumen des Führungsrohres 10 bildet einen Führungskanal 14 und erweitet sich an dem offenen proximalen Ende zu einem Einführkonus 16. Distal ist der Führungskanal 14 zu einer seitlich in der Wandung des Führungsrohres 10 ausgebildeten Öffnung 18 geführt. Die Öffnung 18 befindet sich hinter dem sich verjüngenden distalen Ende 12 des Führungsrohres 10 in dessen Zylindermantelfläche. Im Inneren des Führungsrohres 10 ist eine Ablenkeinrichtung 20 ausgebildet, die in Form einer keilförmigen Schräge oder einer bogenförmig gerundeten Rampe einen stufenlosen Übergang des koaxialen Führungskanals 14 in die seitliche Öffnung 18 bildet. Das Führungsrohr 10 ist vorzugsweise aus Edelstahl gefertigt, kann gegebenenfalls aber auch aus einem geeigneten Kunststoff hergestellt werden.

Das Führungsrohr 10 ist drehbar und lösbar an einem Griffteil 22 angebracht. Hierzu weist das proximale Ende des Führungsrohres 10 einen Außenbund 24 auf, der einen gegenüber dem kreisförmigen Querschnitt des Führungsrohres 10 im Durchmesser vergrößerten kreisförmigen Querschnitt aufweist. Der Außenbund 24 ist vorzugsweise einstückig an dem Führungsrohr 10 angeformt. In die zylindrische Außenmantelfläche des Außenbundes 24 ist eine radiale Sackbohrung 26 mit einem Innengewinde eingearbeitet. Der Außenbund 24 wird in einen Lagerring 28 des Griffteils 22 eingesetzt. Der Innendurchmesser des Lagerrings 28 entspricht dem Außendurchmesser des Außenbundes 24, so dass der Außenbund 24 in dem Lagerring 28 drehbar geführt gelagert ist. Die axiale Länge des Außenbundes 24 entspricht der axialen Länge des Lagerringes 28. Der Lagerring 28 weist einen radial durchgehenden und in Umfangsrichtung verlaufenden Umfangsschlitz 30 auf, der sich über nahezu 360° des Umfangs des Außenbundes 24 erstreckt. In einfacher Weise kann der Umfangsschlitz 30 dadurch hergestellt werden, dass der Lagerring 28 aus zwei um die Breite des Umfangsschlitzes 30 axial beabstandeten Einzelringen zusammengesetzt ist, die durch einen axialen Steg 32 in einem engen Winkelabschnitt miteinander verbunden sind. Der Außenbund 24 wird axial in den Lagerring 28 eingeschoben, worauf sich die Sackbohrung 26 in dem axialen Bereich des Umfangsschlitzes 30 befindet. Dann wird ein als Betätigungshebel dienender radialer Stift 34 durch den Umfangsschlitz 30 eingesetzt und in die Sackbohrung 26 eingeschraubt. Der Stift 34 ragt radial über den Lagerring 28 hinaus, so dass der Stift 34 leicht erfasst werden kann, um den Außenbund 24 und damit das Führungsrohr 10 gegenüber dem Lagerring 28 und damit dem Griffteil 22 zu verdrehen. Eine Drehung des Führungsrohres 10 ist damit um einen Winkel möglich, der dem Umfangswinkel des Umfangsschlitzes 30 entspricht, d. h. um einen Winkel von nahezu 360°. Die Sackbohrung 26 und damit der Stift 34 sind vorzugsweise in der selben Winkelposition des Führungsrohres 10 angeordnet, in welcher sich die Öffnung 18 befindet. Der Stift 34 zeigt somit auch die Winkelstellung der Öffnung 18 in Bezug auf das Griffteil 22 an.

Der in die Sackbohrung 26 eingeschraubte Stift 34 hält den Außenbund 24 und damit das Führungsrohr 10 axial in dem Lagerring 28 und damit an dem Griffteil 22. Durch Herausdrehen des Stiftes 34 aus der Sackbohrung 26 kann das Führungsrohr 10 wieder von dem Griffteil 22 getrennt werden.

Das Griffteil 22 weist weiter eine Aufnahme 36 auf, die als Handgriff ausgebildet ist und zum Führen des Instruments dient. Die Aufnahme 36 hat die Form eines länglichen hohlen Kreiszylinders und weist an ihrem Außenumfang Umfangsrillen auf, die die Griffigkeit der Aufnahme 36 als Handgriff begünstigen. Am distalen Ende der Aufnahme 36 ist der achsparallele Steg 32 angebracht, der an seinem der Aufnahme 36 entgegengesetzten distalen Ende den Lagerring 28 bzw. die zwei den Lagerring 28 bildenden Einzelringe trägt. Am proximalen Ende der Aufnahme 36 sind zwei Anschlagbügel 38 angebracht. Die zwei Anschlagbügel 38 sind im Winkel gegeneinander versetzt an der proximalen Stirnkante der Aufnahme 36 angebracht, verlaufen von der Aufnahme 36 achsparallel in proximale Richtung und sind an ihrem freien proximalen Ende rechtwinklig nach innen abgebogen, um jeweils einen kurzen Anschlag 40 zu bilden.

Wird das Instrument zum Injizieren verwendet, so wird in das Instrument eine Injektionsspritze eingesetzt, insbesondere eine handelsübliche Spritze, z. B. eine Einmal-Spritze. Die Spritze weist einen Zylinder 42 auf, an dessen distalen Kanülenansatz 44 eine Kanüle 46 angesetzt wird. Am proximalen Ende des Zylinders 42 ist ein seitlich abstehender Flansch 48 angeformt. In dem Zylinder 42 ist ein Kolben 50 verschiebbar.

Die Spritze wird mit aufgesetzter Kanüle 46 vom proximalen Ende durch die Aufnahme 36 in das Instrument eingeführt. Die Kanüle 46 gelangt dabei begünstigt durch den Einführkonus 16 in den Führungskanal 14 des Führungsrohres 10. Der Zylinder 42 der Spritze wird koaxial in der Aufnahme 36 aufgenommen. Die Spritze wird soweit in das Instrument eingeführt, bis sich der Flansch 48 am proximalen Ende des Zylinders 42 zwischen dem proximalen Ende der Aufnahme 36 und den Anschlägen 40 der Anschlagbügel 38 befindet. Die Anschläge 40 greifen dabei beiderseits des Kolbens 50 über den Flansch 48.

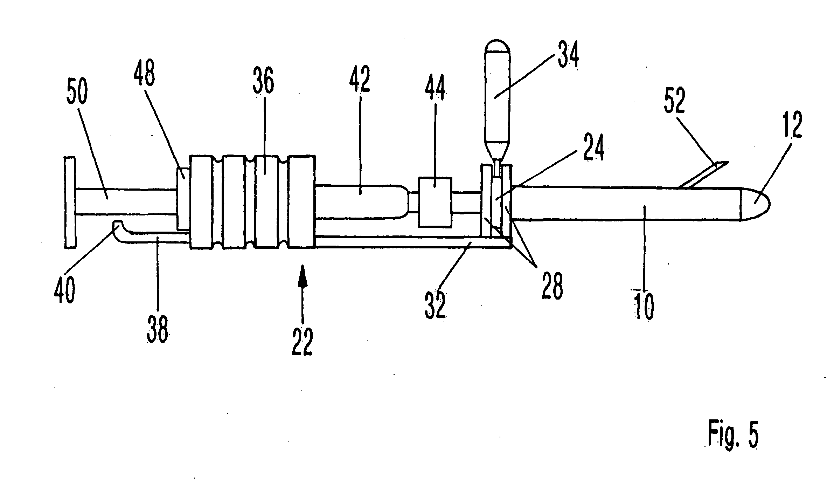

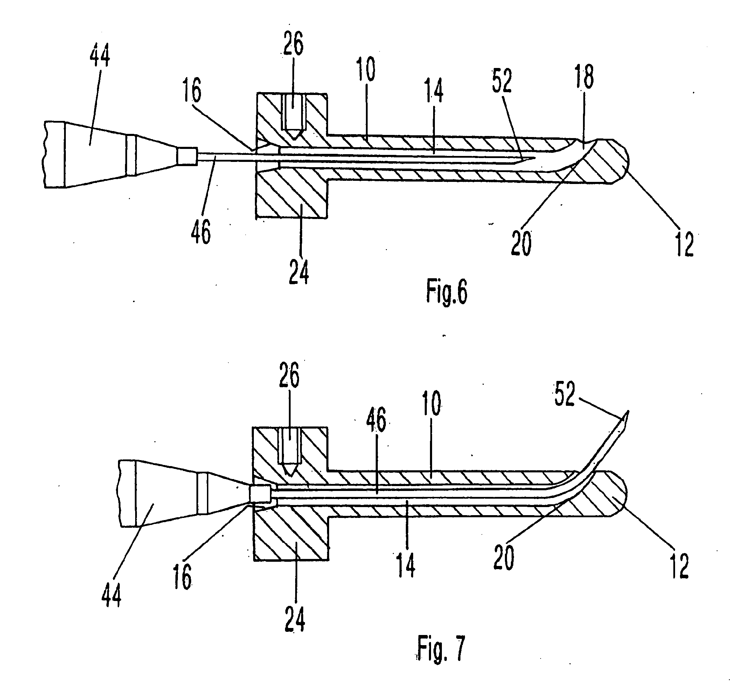

Die axialen Längenabmessungen des Instruments sind auf die axialen Längenabmessungen der Spritze, d. h. insbesondere des Zylinders 42 und der Kanüle 46, so abgestimmt, dass sich die distale Spitze 52 der Kanüle 46 im Inneren des Führungskanals 14 hinter der Ablenkeinrichtung 20 befindet, wie Figur 6 zeigt, wenn der Zylinder 42 mit seinem Flansch 48 an den Anschlägen 40 der Anschlagbügel 38 anliegt. Da die scharfe distale Spitze 52 der Kanüle 46 im Inneren des Führungsrohres 10 liegt, kann das Führungsrohr in den Körperkanal des Patienten eingeführt werden, ohne dass Verletzungen durch die Kanüle 46 möglich sind. Ist das Führungsrohr 10 axial an der Injektionsstelle platziert, so wird der Zylinder 42 der Spritze distal in der Aufnahme 36 vorgeschoben, bis der Flansch 48 an dem proximalen Ende der Aufnahme 36 anschlägt, wie dies in Figur 5 dargestellt ist. Dabei wird die Kanüle 46 in dem Führungskanal 14 ebenfalls distal vorgeschoben, wobei die scharfe Spitze 52 der Kanüle 46 durch die Ablenkeinrichtung 20 abgelenkt wird und seitlich durch die Öffnung 18 aus dem Führungsrohr 10 austritt, wie dies in Figur 7 dargestellt ist. Der Anschlag des Flansches 48 an der Aufnahme 36 definiert dadurch auch, wie weit die Spitze 52 der Kanüle 46 aus dem Führungsrohr 10 austritt, wodurch die Einstichtiefe der Kanüle 46 für die Injektion festgelegt ist. Bei dieser Injektion hält der behandelnde Arzt das Instrument an der als Handgriff ausgebildeten Aufnahme 36. Das Führungsrohr 10 kann mittels des Stiftes 34 koaxial gegenüber dem Griffteil 22 gedreht werden, so dass der behandelnde Arzt bei festgehaltenem Griffteil 22, d. h. bei definierter axialer Position der Öffnung 18 durch Drehen des Stiftes 34 die Winkelposition der Öffnung 18 festlegen kann und damit die Winkelposition, in welcher die Spitze 52 der Kanüle 46 aus dem Führungsrohr 10 austritt und in die Wand des Körperkanals eindringt. Den Einstichwinkel der Spitze 52 in Bezug auf die Achse des Führungsrohres 10 bestimmt dabei die Ablenkeinrichtung 20. Nach einer Injektion in einer ersten Drehwinkelstellung kann die Spritze wieder bis an die Anschläge 40 zurückgezogen werden, wodurch die Spitze 52 der Kanüle 46 wieder in den Führungskanal 14 hineingezogen wird. Das Führungsrohr 10 kann nun in eine neue Winkelposition gedreht werden, um dann durch erneutes distales Verschieben der Spritze wiederum die Spitze 52 in einer anderen Winkelposition einzustechen und erneut zu injizieren.

Es ist offensichtlich, dass die Aufnahme 36 nicht selbst als Handgriff ausgebildet sein muss. Es ist ebenso möglich, an der Aufnahme 36 einen zusätzlichen radial abstehenden Handgriff anzubringen, z. B. nach Art eines Pistolengriffes. Die Ausbildung der Aufnahme 36 als Handgriff hat den Vorteil, dass das Instrument geringe radiale Abmessungen aufweist, was in vielen Fällen die Handhabung erleichtert.

Weiter ist es möglich, Rastungen vorzusehen, die ein federndes Einrasten des Führungsrohres 10 gegenüber dem Griffteil 22 in vorgegebenen definierten Drehwinkelpositionen ermöglicht. Dies erleichtert die Drehwinkelpositionierung beim Einstechen der Kanüle 46 in die Wand des Körperkanals. In der Regel ist jedoch die Reibung zwischen dem Außenbund 24 und dem Lagerring 28 ausreichend, um das Führungsrohr 10 in der jeweils gewählten Drehwinkelposition zu halten, wobei sich hierbei der Vorteil einer kontinuierlichen Winkelverstellung ergibt.

### Bezugszeichenliste

- 10: Führungsrohr
- 12: distales Ende
- 14: Führungskanal
- 16: Einführkonus
- 18: Öffnung
- 20: Ablenkeinrichtung
- 22: Griffteil
- 24: Außenbund
- 26: Sackbohrung
- 28: Lagerring
- 30: Umfangsschlitz
- 32: Steg
- 34: Stift
- 36: Aufnahme
- 38: Anschlagbügel
- 40: Anschlag
- 42: Zylinder
- 44: Kanülenansatz
- 46: Kanüle
- 48: Flansch
- 50: Kolben
- 52: Spitze

## Patentansprüche

1. Medizinisches Instrument mit einem Führungsrohr (10), das in einen Körperkanal eines Patienten einführbar ist, wobei das Führungsrohr (10) einen inneren axialen Führungskanal (14) aufweist, der hinter dem geschlossenen distalen Ende (12) des Führungsrohres (10) in eine seitlich am Umfang des Führungsrohres (10) angeordnete Öffnung (18) mündet, so dass eine elastisch biegsame Kanüle (46) proximal in den Führungskanal (14) einführbar ist und beim Vorschieben in dem Führungskanal (14) mit ihrer distalen Spitze (52) gegen die Achse des Führungsrohres (10) abgebogen seitlich durch die Öffnung (18) austritt,
**dadurch gekennzeichnet, dass** das Führungsrohr (10) mit seinem proximalen Ende um seine Längsachse drehbar in einem Griffteil (22) aufgenommen ist.

2. Instrument nach Anspruch 1,
**dadurch gekennzeichnet, dass** das Führungsrohr (10) lösbar in dem Griffteil (22) aufgenommen ist.

3. Instrument nach Anspruch 1 oder 2,
**dadurch gekennzeichnet, dass** eine Markierung die jeweilige Drehwinkelposition des Führungsrohres (10) gegenüber dem Griffteil (22) erkennbar macht.

4. Instrument nach einem der vorhergehenden Ansprüche,
**dadurch gekennzeichnet, dass** das Führungsrohr (10) an seinem proximalen Ende einen im Wesentlichen radial abstehenden Betätigungshebel (Stift 34) aufweist.

5. Instrument nach einem der vorhergehenden Ansprüche,
**dadurch gekennzeichnet, dass** das Führungsrohr (10) an seinem proximalen Ende einen Außenbund (24) aufweist, der in einem Lagerring (28) am distalen Ende des Griffteiles (22) drehbar gelagert ist.

6. Instrument nach Anspruch 5,
**dadurch gekennzeichnet, dass** der Lagerring (28) einen Umfangsschlitz (30) aufweist, durch welchen der Betätigungshebel (Stift 34) radial hindurch tritt.

7. Instrument nach Anspruch 6,
**dadurch gekennzeichnet, dass** der Betätigungshebel (Stift 34) in den Außenbund (24) lösbar eingesetzt ist.

8. Instrument nach einem der vorhergehenden Ansprüche,
**dadurch gekennzeichnet, dass** das Griffteil (22) eine Aufnahme (36) für eine Spritze aufweist, in welcher die Spritze axial bewegbar ist zwischen einer proximalen Position, in welcher sich die distale Spitze (52) ihrer Kanüle (46) zurückgezogen in dem Führungskanal (14) befindet, und einer distalen Position, in welcher die distale Spitze (52) der Kanüle (46) durch die Öffnung (18) aus dem Führungsrohr (10) austritt.

9. Instrument nach Anspruch 8,
**dadurch gekennzeichnet, dass** die Aufnahme (36) einen Anschlag aufweist, der die axiale Bewegung der Spritze in distaler Richtung begrenzt.

10. Instrument nach Anspruch 8 oder 9,
**dadurch gekennzeichnet, dass** die Aufnahme (36) einen Anschlag (38, 40) aufweist, der die axiale Bewegung der Spritze in proximaler Richtung begrenzt.

11. Instrument nach einem der Ansprüche 8 bis 10,
**dadurch gekennzeichnet, dass** das Griffteil (22) eine hohlzylindrische Aufnahme (36) aufweist, die den Zylinder (42) der eingesetzten Spritze koaxial umschließt.

12. Instrument nach Anspruch 11,
**dadurch gekennzeichnet, dass** die Aufnahme (36) als Handgriff ausgebildet ist.

13. Instrument nach Anspruch 11,
**dadurch gekennzeichnet, dass** an der Aufnahme (36) ein Handgriff angebracht ist.

14. Instrument nach einem der Ansprüche 5 bis 13,
**dadurch gekennzeichnet, dass** der Lagerring (28) an der Aufnahme (36) befestigt ist.

15. Instrument nach einem der Ansprüche 9 bis 14,
**dadurch gekennzeichnet, dass** das proximale Ende der Aufnahme (36) den distalen Anschlag für einen Flansch (48) der Spritze bildet.

16. Instrument nach einem der Ansprüche 10 bis 15,
**dadurch gekennzeichnet, dass** an der Aufnahme (36) ein proximal abstehender Anschlag (38, 40) für den Flansch (48) der Spritze als proximaler Anschlag angebracht ist.

## Claims

1. A medical instrument with a guide tube (10) which is capable of being inserted into a duct in the body of a patient, wherein the guide tube (10) has an internal axial guide channel (14) which opens behind the closed distal end (12) of the guide tube (10) into an opening (18) arranged laterally on the periphery of the guide tube (10), so that a resiliently flexible cannula (46) is capable of being inserted proximally into the guide channel (14) and during the advance in the guide channel (14) emerges in a curved manner laterally through the opening (18) with its distal tip (52) towards the axis of the guide tube (10), **characterized in that** the guide tube (10) is received with its proximal end in a gripping part (22) so as to be rotatable about its longitudinal axis.

2. An instrument according to claim 1, **characterized in that** the guide tube (10) is received in the gripping part (22) in a releasable manner.

3. An instrument according to claim 1 or 2, **characterized in that** a marking indicates the respective rotational angle position of the guide tube (10) with respect to the gripping part (22).

4. An instrument according to any one of the preceding claims, **characterized in that** at its proximal end the guide tube (10) has an actuating lever (pin 34) projecting substantially radially.

5. An instrument according to any one of the preceding claims, **characterized in that** at its proximal end the guide tube (10) has an external collar (24) which is mounted in a rotatable manner in a bearing ring (28) at the distal end of the gripping part (22).

6. An instrument according to claim 5, **characterized in that** the bearing ring (28) has a peripheral slot (30) through which the actuating lever (pin 34) passes radially.

7. An instrument according to claim 6, **characterized in that** the actuating lever (pin 34) is inserted into the external collar (24) in a releasable manner.

8. An instrument according to any one of the preceding claims, **characterized in that** the gripping part (22) has a receiving means (36) for a syringe, in which the syringe is movable axially between a proximal position, in which the distal tip (52) of its cannula (46) is arranged retracted in the guide channel (14), and a distal position, in which the distal tip (52) of the cannula (46) passes through the opening (18) out of the guide tube (10).

9. An instrument according to claim 8, **characterized in that** the receiving means (36) has a stop which limits the axial movement of the syringe in the distal direction.

10. An instrument according to claim 8 or 9, **characterized in that** the receiving means (36) has a stop (38, 40) which limits the axial movement of the syringe in the proximal direction.

11. An instrument according to any one of claims 8 to 10, **characterized in that** the gripping part (22) has a hollow cylindrical receiving means (36) which surrounds the cylinder (42) of the inserted syringe in a coaxial manner.

12. An instrument according to claim 11, **characterized in that** the receiving means (36) is designed in the form of a handle.

13. An instrument according to claim 11, **characterized in that** a handle is attached to the receiving means (36).

14. An instrument according to any one of claims 5 to 13, **characterized in that** the bearing ring (28) is fastened to the receiving means (36).

15. An instrument according to any one of claims 9 to 14, **characterized in that** the proximal end of the receiving means (36) forms the distal stop for a flange (48) of the syringe.

16. An instrument according to any one of claims 10 to 15, **characterized in that** a stop (38, 40) projecting in a proximal manner for the flange (48) of the syringe is attached as a proximal stop to the receiving means (36).

## Revendications

1. Instrument médical comportant un tube de guidage (10) pouvant être introduit dans un canal corporel d'un patient, ce tube de guidage (10) comportant un canal de guidage axial interne (14) qui débouche en arrière de l'extrémité distale fermée (12) du tube de guidage (10) par une ouverture (18) située latéralement à la périphérie de ce tube (10) de façon à permettre l'introduction d'une canule (46) flexible élastiquement à l'extrémité proximale du canal de guidage (14), et en la poussant dans ce canal de guidage (14) la sortie, par l'ouverture (18) de son extrémité distale en forme de pointe (52) latéralement recourbée par rapport à l'axe du tube de guidage (10),
**caractérisé en ce que**
l'extrémité proximale du tube de guidage (10) est montée rotative autour de l'axe longitudinal de ce tube dans un élément de manipulation (22).

2. Instrument conforme à la revendication 1,
**caractérisé en ce que**
le tube de guidage (10) est monté amovible dans l'élément de manipulation (22).

3. Instrument conforme à la revendication 1 ou 2,
**caractérisé en ce que**
un marquage permet de visualiser la position angulaire respective du tube de guidage (10) par rapport à l'élément de manipulation (22).

4. Instrument conforme à l'une des revendications précédentes,
**caractérisé en ce que**
le tube de guidage (10) est équipé, à son extrémité proximale d'un levier de commande (broche 34) s'étendant essentiellement radialement.

5. Instrument conforme à l'une des revendications précédentes,
**caractérisé en ce que**
le tube de guidage (10) est équipé à son extrémité proximale d'une collerette externe (24) qui est montée mobile en rotation dans une bague formant palier (28) située à l'extrémité distale de l'élément de manipulation (22).

6. Instrument conforme à la revendication 5,
**caractérisé en ce que**
la bague formant palier (28) comporte une fente périphérique (30) dans laquelle le levier de commande (broche 34) pénètre radialement.

7. Instrument conforme à la revendication 6,
**caractérisé en ce que**
le levier de commande (broche 34) est logé amovible dans la collerette externe (24).

8. Instrument conforme à l'une des revendications précédentes,
**caractérisé en ce que**
l'élément de manipulation (22) comporte un organe de réception (36) d'une seringue, dans lequel cette seringue est mobile axialement entre une position proximale dans laquelle la pointe distale (52) de sa canule (46) est rétractée dans le canal de guidage (14) et une position distale dans laquelle la pointe distale (52) de la canule (46) sort du tube de guidage (10) par l'ouverture (18) de ce tube.

9. Instrument conforme à la revendication 8,
**caractérisé en ce que**
l'organe de réception (36) comporte une butée qui limite le mouvement axial de la seringue en direction distale.

10. Instrument conforme à la revendication 8 ou 9,
**caractérisé en ce que**
l'organe de réception (36) comporte une butée (38, 40) qui limite le mouvement axial de la seringue en direction proximale.

11. Instrument conforme à l'une des revendications 8 à 10,
**caractérisé en ce que**
l'élément de manipulation (22) comporte un organe de réception cylindrique creux (36) qui entoure co-axialement le cylindre (42) de la seringue introduite dans cet élément.

12. Instrument conforme à la revendication 11,
**caractérisé en ce que**
l'organe de réception (36) est réalisé sous la forme d'une poignée.

13. Instrument conforme à la revendication 11,
**caractérisé en ce qu'**
une poignée est montée sur l'organe de réception (36).

14. Instrument conforme à l'une des revendications 5 à 13,
**caractérisé en ce que**
la bague formant palier (28) est fixée sur l'organe de réception (36).

15. Instrument conforme à l'une des revendications 9 à 14,
**caractérisé en ce que**
l'extrémité proximale de l'organe de réception (36) forme une butée distale pour une bride (48) de la seringue.

16. Instrument conforme à l'une des revendications 10 à 15,
**caractérisé en ce que**
une butée (38, 40) pour la bride (48) de la seringue s'étendant en direction proximale et faisant office de butée proximale est montée sur l'organe de réception (36).
